Europäisches Patentamt

European Patent Office

Office européen des brevets

(11) Publication number: **0 117 731**
A1

## (12) EUROPEAN PATENT APPLICATION

(21) Application number: 84301189.1

(22) Date of filing: 23.02.84

(51) Int. Cl.³: **C 07 C 19/02**, C 07 C 17/154

(30) Priority: 26.02.83 GB 8305406

(43) Date of publication of application: 05.09.84
Bulletin 84/36

(84) Designated Contracting States: **BE DE FR GB IT NL SE**

(71) Applicant: **The British Petroleum Company p.l.c.,
Britannic House Moor Lane, London EC2Y 9BU (GB)**

(72) Inventor: **Bromhead, Jillian, The British Petroleum
Company p.l.c. Chertsey Road, Sunbury-on-Thames
Middlesex, TW16 7LN (GB)**
Inventor: **Font-Freide, Josephus Johannes H. M., The
British Petroleum Company p.l.c. Chertsey Road,
Sunbury-on-Thames Middlesex, TW16 7LN (GB)**
Inventor: **Westlake, David Jack, The British Petroleum
Company p.l.c. Chertsey Road, Sunbury-on-Thames
Middlesex, TW16 7LN (GB)**

(74) Representative: **Harry, John et al, c/o The British
Petroleum Company plc Patents Division Chertsey Road,
Sunbury-on-Thames Middlesex TW16 7LN (GB)**

(54) Process for the production of methyl or ethyl mono-chloride or bromide.

(57) Methyl or ethyl mono-chloride or bromide are produced
selectively from methane or ethane and hydrogen chloride or
bromide by feeding the methane or ethane and hydrogen chloride or bromide and a source of molecular oxygen to an oxychlorination zone maintained at a temperature below 450°C
and containing a supported oxychlorination catalyst.

EP 0 117 731 A1

1

## PROCESS FOR THE PRODUCTION OF METHYL
## OR ETHYL MONO-CHLORIDE OR BROMIDE

The present invention relates to a process for the production of methyl or ethyl mono-chloride or bromide.

Methyl mono-halides, being functional derivatives of methane, are potentially important intermediates in the production, for example of methanol. Converting methane to methyl mono-halides and thence to methanol offers an alternative to converting it first to synthesis gas (carbon monoxide + hydrogen) and thereafter to methanol. Similarly, ethyl mono-halides, being functional derivatives of ethane, are potentially important intermediates in the production, for example of ethanol. Converting ethane to ethyl mono-halides and thence to ethanol offers an alternative to converting it first to ethylene and thereafter to ethanol.

It is known to produce such alkyl mono-halides by reacting methane or ethane with a halogen, but this is conventionally carried out at elevated temperatures at which the selectivity to the desired alkyl mono-halides is low, there being generally formed in addition to the alkyl mono-halide a variety of halogenated alkane derivatives. Thus in the case of chlorine as the halogen for example the reaction is usually carried out at temperatures above 450°C at which temperatures in addition to methyl chloride substantial amounts of methylene dichloride, chloroform and carbon tetrachloride are also obtained.

It is also well-known to produce chlorine from hydrogen chloride by oxidation with air or oxygen at temperatures in the region of 400°C or above using a suitable catalyst, such as cupric

1

chloride, either supported on an inert support or in the form of a melt together with a melting point depressant, such as potassium chloride. This process is generally referred to as the Deacon process.

Although the Deacon process has been largely overtaken by electrolytic processes for the production of chlorine, it is still utilised with simultaneous chlorination of organics in the production, for example, of 1,2-dichloroethane by oxychlorination of ethylene.

It has now been found unexpectedly that methyl or ethyl mono-chloride or bromide can be obtained in high selectivities by oxyhalogenating methane or ethane at temperatures below 450°C.

Accordingly, the present invention provides a process for the selective production of methyl or ethyl mono-chloride or bromide which process comprises feeding methane or ethane and hydrogen chloride or bromide and a source of molecular oxygen to an oxychlorination zone maintained at a temperature below 450°C and containing a supported oxychlorination catalyst.

By the term "selective production" as used throughout this specification is meant, in the case of the production of the monohalide from methane that 80% or more of the converted methane is in the form of the monohalide and, in the case of the production of the monohalide from ethane that 60% or more of the converted ethane is in the form of the monohalide. Generally, in the case of methane, the remaining 20% or less of the converted methane is in the form of multihalogenated methane. However, in the case of ethane the remaining 40% or less of the converted ethane may comprise products other than multihalogenated ethanes.

Both methane and ethane are abundantly available in natural gas reservoirs. Methane may also be obtained by the biological conversion of organic materials and by the methanation or in-situ gasification of coal.

Hydrogen chloride is available in vast amounts as a by-product from other integrated chlorination processes, such as for example the production of chlorinated solvents and synthetic resins.

Although not available on such an extensive scale, hydrogen bromide is nevertheless readily obtainable on a commercial scale.

A preferred source of molecular oxygen is air, though gases richer or poorer in oxygen than air, for example molecular oxygen, may be employed.

Suitable oxychlorination catalysts include those comprising compounds of one or more multivalent metals. Suitable multivalent metals include copper, nickel, iron and palladium and suitable compounds include the halide salts of the metal. Other metallic salts or compounds may also be incorporated in the catalyst if desired. Suitable metallic salts include alkali metal halides.

Preferably the metallic compounds are deposited on a support. Suitable supports include silica, alumina, silica/alumina, natural and synthetic zeolites, clays, pillared clays and the like. The metal may be deposited on the support by any suitable technique such as impregnation, precipitation, co-precipitation etc. The metals may be present on the support in any suitable amount, preferably from about 0.1 to about 20% w/w more preferably from 0.5 to 5% w/w.

The proportions of methane or ethane, hydrogen chloride or bromide and molecular oxygen-containing gas in the feed may be varied over a wide range. Suitably, the molar ratio of methane or ethane to hydrogen chloride or bromide may be in the range from 0.5 to 10, preferably from 1 to 4. The molar ratio of methane or ethane to molecular oxygen-containing gas may suitably be in the range from 0.1 to 5, preferably from 0.5 to 2.

The oxychlorination zone is maintained at a temperature below 450°C, preferably below 375°C, even more preferably at or below 350°C, and suitably as low as 250°C. Preferably the zone is maintained at a temperature in the range from 300 to 350°C. The specific temperatures selected within the aforesaid ranges will depend to some extent on the nature of the particular oxychlorination catalyst which is to be employed, for example using iron-based catalysts temperatures in the upper parts of the aforesaid ranges may be employed. Thus, using iron-based catalyst temperatures up to 450°C may be used. However, using other catalysts, for example copper-based catalysts, lower temperatures within the aforesaid ranges are desirable.

The products of the process may be recovered and separated by methods known in the art. It is preferred to produce methyl or ethyl chloride by feeding methane or ethane, hydrogen chloride and air to the oxychlorination zone.

The process may be operated batchwise or continuously, preferably continuously.

The invention will now be illustrated by reference to the following Examples.

Example 1

A mixture of methane, hydrogen chloride and oxygen (mole ratio 4:2:1) was passed over copper chloride impregnated alumina (2.9% wt Cu) in a continuous flow reactor (contact time 13 sec) which was heated by means of an electric furnace. The heated zone of the catalyst bed was at 325°C. The product stream was analysed by gas chromatography.

The composition of the product stream, excluding unreacted reactants, was methyl chloride (88% v/v) and methylene chloride (12% v/v) and the methane conversion was 19%.

Example 2

A mixture of methane, hydrogen chloride and air (volume ratio 4:2:5) was passed over ferric chloride impregnated celite (0.8% wt Fe) in a continuous flow reactor (contact time 46 sec). The reactor was heated by means of an electric furnace and the heated zone of the catalyst bed was at 350°C. The product stream was analysed by gas chromatography.

The product stream, excluding unreacted feed gases, was methyl chloride (greater than 99%) and the methane conversion was 23%.

Comparative Test 1

The experiment described in Example 2 above was repeated under similar conditions. The heated zone of the catalyst bed was maintained at 500°C.

The composition of the gaseous product stream, excluding unreacted reactants, was methyl chloride (63% v/v) and methylene chloride (37% v/v) with a methane conversion of 32%.

Comparative Test 2

Comparative Test 1 was repeated under similar conditions with copper chloride impregnated celite. The heated zone of the catalyst bed was maintained at 450°C.

The composition of the gaseous product stream, excluding unreacted reactants, was methyl chloride (58% v/v), methylene chloride (40% v/v) and chloroform (2% v/v) and the methane conversion was 27%.

These comparative tests are presented to demonstrate that at elevated temperatures (450°C and above) conversions are obtained with low selectivity to methyl chloride and increased selectivity to multichlorinated methanes. These are not examples according to the invention and they are only included for the purpose of comparison.

Example 3

A mixture of ethane, hydrogen chloride and air (volume ratio 9:2:5) was passed over copper chloride impregnated alumina (2.9% wt Cu) in a continuous flow reactor (contact time 7 sec). The reactor was heated by means of an electric furnace and the heated zone of the catalyst bed was at 325°C. The product stream was analysed by gas chromatography.

The composition of the product stream, excluding unreacted feed gases, was ethyl chloride (67% v/v), vinyl chloride (15% v/v) and 1,2-dichloroethane (18% v/v) with an ethane conversion of 13%.

6

Claims:

1. A process for the selective production of methyl monochloride or monobromide from methane which process comprises feeding methane and either hydrogen chloride or hydrogen bromide and a source of molecular oxygen to an oxychlorination zone maintained at a temperature below 450°C and containing a supported oxychlorination catalyst.

2. A process according to claim 1 wherein the molar ratio of methane to hydrogen chloride or bromide is in the range from 1 to 4 and the molar ratio of methane to molecular oxygen-containing gas is in the range from 0.5 to 2.

3. A process for the selective production of ethyl monochloride or monobromide from ethane which process comprises feeding ethane and either hydrogen chloride or hydrogen bromide and a source of molecular oxygen to an oxychlorination zone maintained at a temperature below 450°C and containing a supported oxychlorination catalyst.

4. A process according to claim 3 wherein the molar ratio of ethane to hydrogen chloride or bromide is in the range from 1 to 4 and the molar ratio of ethane to molecular oxygen-containing gas is in the range from 0.5 to 2.

5. A process according to any one of the preceding claims wherein the oxychlorination catalyst is a compound of one or more of the metals copper, nickel, iron or palladium.

6. A process according to claim 5 wherein the compound is a halide salt.

7. A process according to either claim 5 or claim 6 wherein the compounds are deposited on a support.

**8.** A process according to any one of the preceding claims wherein the temperature is below 375°C.

**9.** A process according to claim 8 wherein the temperature is in the range from 300 to 350°C.

**10.** A process according to claim 1 wherein methane, hydrogen chloride and a source of molecular oxygen-containing gas are fed to the oxychlorination zone.

0117731

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | CHEMICAL ABSTRACTS, vol. 82, no. 17, 28th April 1975, page 468, no. 111555x, Columbus, Ohio, US & SU - A - 446 497 (V.M. VLASENKO et al.) 15-10-1974 * Abstract * | 1,8-10 | C 07 C 19/02<br>C 07 C 17/154 |
| X | GB-A- 938 096 (PITTSBURGH PLATE GLASS COMPANY) * Table III; claims * | 3-9 | |
| X | GB-A-1 274 150 (ASAHI GLASS COMPANY) * Examples * | 1,2,5-7,10 | |

|  | TECHNICAL FIELDS SEARCHED (Int. Cl. ³) |
|---|---|
|  | C 07 C 17/00<br>C 07 C 19/00 |

The present search report has been drawn up for all claims

| Place of search | Date of completion of the search | Examiner |
|---|---|---|
| THE HAGUE | 07-05-1984 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document